# EUROPEAN PATENT APPLICATION

(11) **EP 4 331 606 A1**
(43) Date of publication of application: **06.03.2024**
(21) Application number: 22795900.4
(22) Date of filing: 28.04.2022
(51) Int. Cl.: A61K 45/00, A61K 31/7088, A61K 31/7105, A61K 31/712, A61K 48/00, A61P 21/00, A61P 21/04, A61P 43/00, C12N 15/113

(54) **PREVENTION OR TREATMENT OF MYOPATHY USING MIR-33B INHIBITOR**

(30) Priority: 30.04.2021 JP 2021077723
(71) Applicant: Kyoto University, Kyoto-shi, Kyoto 606-8501 (JP); OSAKA UNIVERSITY, Suita-shi Osaka 565-0871 (JP)
(72) Inventor: KOTERA, Jun, Osaka-shi, Osaka 541-8505 (JP); ONO, Koh, Kyoto-shi, Kyoto 606-8501 (JP); HORIE, Takahiro, Kyoto-shi, Kyoto 606-8501 (JP); SOWA, Naoya, Kyoto-shi, Kyoto 606-8501 (JP); IDE, Yuya, Kyoto-shi, Kyoto 606-8501 (JP); OBIKA, Satoshi, Suita-shi, Osaka 565-0871 (JP); KASAHARA, Yuya, Ibaraki-shi, Osaka 567-0085 (JP)
(74) Representative: Gill Jennings & Every LLP
(86) International application number: PCT/JP2022/019329
(87) International publication number: WO 2022/230987

(57) **Abstract**

A prophylactic or therapeutic agent for a myopathy, including a miR-33b inhibitor, preferably an antisense oligonucleotide against miR-33b, as an effective component.

## Description

### TECHNICAL FIELD

The present disclosure relates to a prophylactic or therapeutic agent for a myopathy, comprising a miR-33b inhibitor as an effective component.

### BACKGROUND ART

Examples of the myopathy include: (1) neurogenic muscle atrophies such as spinal muscular atrophy, spinal and bulbar muscular atrophy, and amyotrophic lateral sclerosis; (2) myogenic muscle atrophies such as muscular dystrophies (for example, congenital muscular dystrophy, limb-girdle muscular dystrophy, facioscapulohumeral muscular dystrophy, and myotonic dystrophy), congenital myopathy, inflammatory myopathies (for example, polymyositis, dermatomyositis, and inclusion body myositis), metabolic myopathies (for example, myopathies due to inborn errors of metabolism, and periodic paralysis), and muscle injuries due to endocrine diseases (for example, thyroid diseases and diabetes); and (3) diseases of the neuromuscular junction, such as myasthenia gravis, myasthenic syndrome, and congenital myasthenia (Non-Patent Document 1).

In these myopathies, motor dysfunction due to skeletal muscle damage occurs as a predominant symptom. This leads to decreases in the muscle strength and the muscle function, and hence disturbs daily life. These diseases are often associated with joint contracture/deformity, respiratory embarrassment, myocardial damage, and the like, often leading to death. The neurogenic atrophies can be roughly divided into motor neuron diseases, in which secondary (lower) motor neurons in the spinal cord are damaged, and peripheral neuropathy, in which peripheral nerves are damaged. The myogenic muscle atrophies (myopathies) include muscular dystrophy, in which the muscle itself is damaged, and which is clearly progressive.

Muscular dystrophy is a hereditary myopathy for which not less than 50 causative genes have been elucidated. This disease causes disorders of skeletal muscle, respiratory muscle, and cardiac muscle, to cause movement disorder as a predominant symptom. Progression of the disease state may cause death due to respiratory embarrassment or myocardial damage. The disease has disease types classified based on the causative gene, and reported examples thereof include Duchenne-typeBecker-type sex chromosome recessive inherited muscular dystrophy, which is associated with abnormality of the dystrophin gene; Fukuyama-type or non-Fukuyama-type congenital muscular dystrophy; limb-girdle muscular dystrophy, which is associated with abnormality of a number of genes such as the calpain 3, dysferlin, and myotilin genes; facioscapulohumeral muscular dystrophy, which is associated with abnormality of the DUX4 gene; Emery-Dreifuss muscular dystrophy; myotonic dystrophy, which is associated with the DMPK gene; and others such as oculopharyngeal muscular dystrophy and distal muscular dystrophy. In recent years, development of antisense oligonucleic acid has proceeded for some types of the Duchenne type, which antisense oligonucleotides normalize a particular gene abnormality to convert the Duchenne type to the Becker type. Some of these oligonucleotides are commercially available, and used for treatment of patients. However, because of the diversity of the Duchenne-type dystrophin mutation, such antisense oligonucleotides are not necessarily applicable to all types of Duchenne-type muscular dystrophin, and development of therapeutic agents for the other muscular dystrophies is still in progress. Therefore, the patients to whom the existing drugs are applicable are limited, and no sufficient therapeutic effects have been reported.

The past drug development has aimed at improving muscle function through limited normalization of the function for cases where dystrophin is the causative gene. However, the recovery of muscle function is limited at present, and therefore drugs with which positive recovery of muscle function can be expected are demanded.

Also for other myopathies, for example, for amyotrophic lateral sclerosis, which is a neurogenic muscle atrophy, normalization of the causative gene has been attempted aiming at recovering the neurological function. However, development of pharmaceuticals therefor has been limited from the viewpoint of the presence of the subtypes of amyotrophic lateral sclerosis and the other neurological and muscular diseases as a whole, so that development of novel pharmaceuticals has been demanded.

Thus, since the main effect in the past drug development has been based on suppression of the progression by normalization of the causative genes, it is thought to be important to create therapeutic agents for myopathies based on a mechanism that enables positive improvement of the muscle function.

Examples of diseases and symptoms that cause decreases in the muscle function, other than the myopathies described above, include cachexia, sarcopenia, disuse atrophy, and hereditary spastic paraplegia.

A miRNA (microRNA) is a small, single-stranded RNA which is encoded in the genome, and which undergoes a multi-stage process of generation to finally have a length of 19 to 25 bases. A miRNA directly binds to the 3'-untranslated region of a plurality of target messenger RNAs (mRNAs) to suppress their translation, or to promote their degradation, thereby negatively regulating the target genes. At present, not less than 2,000 kinds of miRNAs have been identified in humans, and it is said that not less than 60% of human gene products undergo certain regulation by miRNAs. Further, since miRNAs are involved in various biological phenomena, and the development and progression of disease conditions, they are attracting attention as biomarkers and novel therapeutic targets.

miR-33 (microRNA-33) was found as a miRNA that targets ATP-binding cassette transporter A1 (ABCA1) to regulate cholesterol metabolism, resulting in a decrease in the high-density lipoprotein cholesterol (HDL-C) level. It has been reported that, while miR-33a and miR-33b are present in humans, only miR-33 (which corresponds to miR-33a in humans) is present in mice (Non-Patent Document 2).

Non-Patent Document 3 discloses that a miR-33a antisense oligonucleotide is applicable to treatment of hereditary spastic paraplegia. However, its action is limited to an action on nerve cells, and involvement of miR-33b in hereditary spastic paraplegia is unclear. Non-Patent Document 4 describes that miR-33a suppresses the growth of myoblasts, that a decrease in miR-33a enhances the growth of myoblasts, and that regulation of miR-33a expression promotes early development of skeletal muscle. However, there is no mention of miR-33b.

Patent Document 1 discloses a method of targeting miRNA molecules for treatment of subjects with diseases, disorders, or conditions. The document mentions miR-33 as one example of candidate target miRNA molecules, and describes amyotrophic lateral sclerosis (ALS) as one example of the diseases. Similarly, Patent Document 2 describes miR-33a and miR-33b as examples of candidate target miRNA molecules, and describes myasthenia gravis (MG) as one example of the diseases (Patent Document 2).

However, none of these documents shows specific data using miR-33b as a therapeutic target, and there is no suggestion on improvement of muscle function by inhibition of miR-33b, or on a prophylactic or therapeutic effect of such improvement on myopathies.

Thus, there is no known document that mentions inhibition of miR-33b that induces improvement of the muscle function based on changes in the expression of genes or based on a differentiation-promoting action on muscle cells, or that mentions treatment and prevention of myopathies represented by muscular dystrophy by such inhibition.

### PRIOR ART DOCUMENTS

### [Patent Documents]

[Patent Document 1] WO2004/076622
[Patent Document 2] WO2012/119051

### [Non-patent Documents]

[Non-Patent Document 1] Homepage of The Japanese Society of Neuropathology
[Non-Patent Document 2] J Am Heart Assoc., 8(13), e012609 (2019).
[Non-Patent Document 3] Clinical science (London, England: 1979), Vol. 133, No. 4, pp. 583-595.
[Non-Patent Document 4] Bioscience reports, (20200626) Vol. 40, No. 6. Journal code: 8102797. E-ISSN: 1573-4935.
[Non-Patent Document 5] Nat Rev Neurol 2019 Jul; 15(7): 373-386.
[Non-Patent Document 6] Acta Neuropathologica volume 134, pages 869-888 (2017).

### SUMMARY OF THE INVENTION

### PROBLEMS TO BE SOLVED BY THE INVENTION

An object of the present invention is to provide a novel prophylactic and/or therapeutic agent for myopathies.

### MEANS FOR SOLVING THE PROBLEMS

The present inventors intensively studied to solve the above problem. More specifically, the inventors focused on the miR-33b molecule, whose association with myopathies has not been known. In order to analyze the contribution of miR-33a and miR-33b to myopathies, studies were carried out using their inhibitors. As a result, miR-33b inhibitors were found to be useful for the treatment, prevention, or amelioration of myopathies, thereby reaching the present invention.

This present disclosure relates to, but is not limited to, the non-limiting, numbered aspects according to the following items [1] to [19].
[1] A prophylactic or therapeutic agent for a myopathy, comprising a miR-33b inhibitor as an effective component.
[2] The prophylactic or therapeutic agent according to [1], wherein the miR-33b inhibitor is a miR-33b-level-reducing substance.
[3] The prophylactic or therapeutic agent according to [2], wherein the miR-33b-level-reducing substance is nucleic acid.
[4] The prophylactic or therapeutic agent according to [3], wherein the miR-33b-level-reducing substance is an antisense oligonucleotide, a siRNA, or a shRNA against miR-33b.
[5] The prophylactic or therapeutic agent according to any one of [2] to [4], wherein the miR-33b-level-reducing substance has a miR-33b-level-reducing rate that is higher than the miR-33a-level-reducing rate.
[6] The prophylactic or therapeutic agent according to any one of [2] to [5], wherein the miR-33b-level-reducing substance is an antisense oligonucleotide against miR-33b.
[7] The prophylactic or therapeutic agent according to [6], wherein the antisense oligonucleotide against miR-33b
   1) is composed of 7 to 20 nucleotide residues, and
   2) has a base sequence which is not less than 90% complementary to an equal length portion of miR-33b having the base sequence of SEQ ID NO:1, wherein no mismatch is present at either the 9th or 10th nucleic acid base as counted from the 5'- end of the miR-33b base sequence of SEQ ID NO:1.
      (SEQ ID NO: 1) GUGCAUUGCUGUUGCAUUGC
[8] The prophylactic or therapeutic agent according to [7], wherein the antisense oligonucleotide against miR-33b has a nucleic acid base sequence having the sequence of AACAGCAATGCA (SEQ ID NO:5) (wherein C is optionally 5-methylcytosine (M)).
[9] The prophylactic or therapeutic agent according to [8], wherein the antisense oligonucleotide is a modified oligonucleotide.
[10] The prophylactic or therapeutic agent according to [9], wherein at least one nucleoside constituting the modified oligonucleotide contains a modified sugar.
[11] The prophylactic or therapeutic agent according to [10], wherein the modified sugar is selected from the sugar moiety of AmNA: 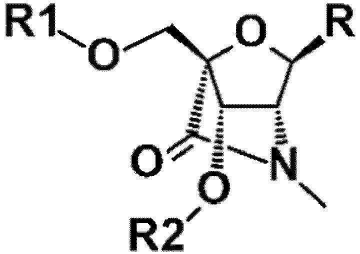 AmNA [wherein R is a nucleic acid base; and R1 and R2 are each independently a phosphate group that is optionally independently substituted].
[12] The prophylactic or therapeutic agent according to [11], wherein the modified oligonucleotide is a modified oligonucleotide of the following formula:
   Aas Ads Mas Ads Gas Cds Aas Ads Tas Gds Mas Ad (SEQ ID NO:8), wherein in the formula,
   each nucleic acid base is represented according to the following symbol: A = adenine, T = thymine, G = guanine, C = cytosine, or M = 5-methylcytosine;
   each sugar moiety is represented according to the following symbol: a = the sugar moiety of AmNA, or d = 2'-deoxyribose; and
   each internucleoside bond is represented according to the following symbol: s = phosphorothioate.
[13] The prophylactic or therapeutic agent according to [12], wherein the modified oligonucleotide is a modified oligonucleotide of the following formula: 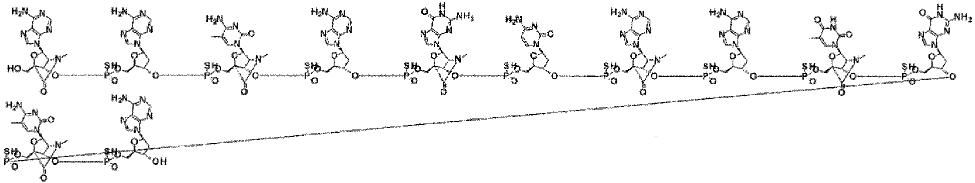
[14] The prophylactic or therapeutic agent according to any one of [1] to [13], which produces a prophylactic or therapeutic effect on a myopathy by increasing the muscle fiber cross-sectional area.
[15] The prophylactic or therapeutic agent according to any one of [1] to [13], which produces a prophylactic or therapeutic effect on a myopathy by promoting muscle differentiation.
[16] The prophylactic or therapeutic agent according to any one of [1] to [15], wherein the myopathy is selected from the group consisting of neurogenic muscle atrophy, myogenic muscle atrophy, and a disease of the neuromuscular junction.
[17] The prophylactic or therapeutic agent according to any one of [1] to [15], wherein the myopathy is selected from the group consisting of cachexia, sarcopenia, disuse atrophy, and hereditary spastic paraplegia.
[18] The prophylactic or therapeutic agent according to [16], wherein the neurogenic muscle atrophy is selected from the group consisting of spinal muscular atrophy, spinal and bulbar muscular atrophy, and amyotrophic lateral sclerosis.
[19] The prophylactic or therapeutic agent according to [16], wherein the myogenic muscle atrophy is selected from the group consisting of muscular dystrophy, congenital myopathy, inflammatory myopathy, and metabolic myopathy.
[20] The prophylactic or therapeutic agent according to [16], wherein the disease of the neuromuscular junction is selected from the group consisting of myasthenia gravis, myasthenic syndrome, and congenital myasthenia.

### EFFECT OF THE INVENTION

Since miR-33b inhibitors suppress occurrence or progression of myopathies, they can be used as prophylactic and/or therapeutic agents for myopathies.

### BRIEF DESCRIPTION OF THE DRAWINGS

Fig. 1A shows photographs of histological staining illustrating a muscle differentiation-promoting action of a miR-33b antisense oligonucleotide in primary skeletal muscle cells derived from miR-33b knock-in/mdx mice.
Fig. 1B shows a graph illustrating a muscle differentiation-promoting action of a miR-33b antisense oligonucleotide in primary skeletal muscle cells derived from miR-33b knock-in/mdx mice.
Fig. 2 shows a graph illustrating the result of an in vivo muscle local administration test for evaluation of the effect of a miR-33a or miR-33b antisense oligonucleotide on the expression level of miR-33a or miR-33b in skeletal muscle of miR-33b knock-in/mdx mice.
Fig. 3 shows graphs illustrating the result of an in vivo muscle local administration test for evaluation of the effect of a miR-33a or miR-33b antisense oligonucleotide on the expression of various genes in skeletal muscle of miR-33b knock-in/mdx mice.
Fig. 4 shows a diagram (partially including photographs) illustrating the result of an in vivo muscle local administration test for evaluation of the effect of a miR-33a or miR-33b antisense oligonucleotide on the muscle cross-sectional area of muscle fibers of miR-33b knock-in/mdx mice.
Fig. 5 shows a diagram (partially including photographs) illustrating the result of an in vivo muscle local administration test for evaluation of the effect of a miR-33a or miR-33b antisense oligonucleotide against muscle fibrosis of a muscle tissue of miR-33b knock-in/mdx mice.
Fig. 6 shows a diagram (partially including photographs) illustrating the result of an in vivo muscle local administration test for evaluation of the effect of a miR-33b antisense oligonucleotide on the expression of Pax7/MyoD in muscle satellite cells of miR-33b knock-in/mdx mice.
Fig. 7 shows a diagram (photographs) illustrating the result of an in vivo muscle local administration test for evaluation of the effect of a miR-33b antisense oligonucleotide on the expression of Utrophin in miR-33b knock-in/mdx mice.
Fig. 8 shows a diagram (photographs) illustrating the result of an in vivo muscle local administration test for evaluation of the effect of a miR-33b antisense oligonucleotide on the protein expression of various genes in skeletal muscle of miR-33b knock-in/mdx mice.

### MODE FOR CARRYING OUT THE INVENTION

### Definition

"Nucleic acid" means a molecule composed of nucleotide units. Examples of the nucleic acid include naturally occurring nucleic acids (ribonucleic acid (RNA) and deoxyribonucleic acid (DNA)) and non-naturally occurring nucleic acids. Examples of the form of the nucleic acid include single-stranded nucleic acid and double-stranded nucleic acid. Examples of functional nucleic acids include small interfering ribonucleic acid (siRNA) and microRNA (miRNA). However, the nucleic acid is not limited to these. A single molecule of nucleic acid may include combination of these elements.

"Nucleic acid base" means a heterocyclic moiety capable of pair formation with another nucleic acid base. The nucleic acid bases include "modified nucleic acid bases" and "unmodified nucleic acid bases".

"Nucleoside" means a nucleic acid base linked to a sugar. In one aspect, a nucleoside is linked to a phosphate group.

"Nucleotide" means a nucleoside containing, for example, a phosphate group covalently bound to the sugar moiety of the nucleoside. A naturally occurring nucleotide contains a sugar moiety that is ribose or deoxyribose, and forms an oligonucleotide or polynucleotide by covalent bonding to a phosphate group through a phosphodiester bond.

"Modified nucleotide" means a nucleotide independently containing a modified sugar moiety, a modified internucleoside bond, or a modified nucleic acid base. "Modified nucleoside" means a nucleoside independently containing a modified sugar moiety or modified nucleic acid base.

"Unmodified nucleotide" means a nucleotide composed of a nucleic acid base, a sugar moiety, and an internucleoside bond that are naturally occurring. In one aspect, an unmodified nucleotide is an RNA nucleotide (that is, a nucleotide containing a β-D-ribonucleoside) or a DNA nucleotide (that is, a nucleotide containing a β-D-deoxyribonucleoside), but the unmodified nucleotide is not limited thereto.

"Internucleoside bond" means a chemical bond between nucleosides.

"Modified internucleoside bond" means a substitution or an arbitrary change from the naturally occurring internucleoside bond (that is, the phosphodiester internucleoside bond). Examples of the modified internucleoside bond include, but are not limited to, a phosphorothioate internucleoside bond.

"Phosphorothioate internucleoside bond" means an internucleoside bond formed by modification of a phosphodiester bond by replacement of one non-crosslinking oxygen atom with a sulfur atom. The phosphorothioate bond is an example of the modified internucleoside bond.

"Modified nucleic acid base" means any nucleic acid base other than adenine, cytosine, guanine, thymidine, or uracil. Examples of the modified nucleic acid base include, but are not limited to, 5-methylcytosine. "Unmodified nucleic acid base" means the purine bases adenine (A) and guanine (G), and the pyrimidine bases thymine (T), cytosine (C), and uracil (U).

"miRNA" means an endogenous non-coding RNA which is produced by cleavage of pre-miR by the enzyme Dicer, and which has a length of 18 to 25 nucleic acid bases. Examples of miRNA can be found in the miRNA database called miRBase (http://microrna.sanger.ac.uk/). In one aspect, miRNA is abbreviated as "miR" or "microRNA".

"Pre-miR" means a non-coding RNA which is produced by cleavage of pri-miR by the double-stranded-RNA-specific ribonuclease called Drosha, and which contains a hairpin structure.

"Stem-loop sequence" means an RNA which contains a hairpin structure, and which has a mature miRNA sequence. In one aspect, the stem-loop sequence is pre-miR. Examples of the stem-loop sequence can be found in the miRNA database called miRBase (http://www.mirbase.org/).

"pri-miR" means a non-coding RNA which is a substrate of the double-stranded-RNA-specific ribonuclease Drosha, and which contains a hairpin structure.

"miRNA precursor" means a transcript which is derived from the genomic DNA, and which contains a non-coding structured RNA containing one or more miRNA sequences. For example, in one aspect, the miRNA precursor is pre-RNA. In one aspect, the miRNA precursor is pri-miR. In one aspect, the miRNA precursor is a stem-loop sequence.

"Direct target mRNA of a miRNA" means a mRNA that is a direct target of a miRNA. The miRNA is fully or almost fully complementary to the target mRNA, and often complementarily binds to the seed sequence (the 7-base sequence corresponding to the second to eighth positions from the 5'-end side of the miRNA), to cause destabilization or translation inhibition of the direct target mRNA.

"Direct target protein of a miRNA" means a protein produced by translation of the direct target mRNA of the miRNA.

"miR-33b stem-loop sequence" means a pre-miR of miRNA-33b having the nucleic acid base sequence

GCGGGCGGCCCCGCGGUGCAUUGCUGUUGCAUUGCACGUGUGUGAGGC
GGGUGCAGUGCCUCGGCAGUGCAGCCCGGAGCCGGCCCCUGGCACCAC
(SEQ ID NO:4).

"miR-33a" means a miRNA having the nucleic acid base sequence GUGCAUUGUAGUUGCAUUGCA (SEQ ID NO:2). (In the present description, each base sequence is described in the order from 5' to 3' .)

"miR-33b" means a miRNA having the nucleic acid base sequence GUGCAUUGCUGUUGCAUUGC (SEQ ID NO: 1).

"miR-33" means a miRNA having the nucleic acid base sequence GUGCAUUGUAGUUGCAUUGCA (SEQ ID NO:3).

In humans, miR-33a and miR-33b are present. In mice, miR-33 is present. Human miR-33a and mouse miR-33 have the same sequence. Therefore, in mice, only miRNA corresponding to human miR-33a is present, and no miRNA corresponding to human miR-33b is present.

"miR-33b seed sequence (also referred to as seed region)" means UGCAUUG in the miR-33b nucleic acid base sequence.

"miR-33a level", "miR-33b level", and "miR-33 level" refer to the abundances (levels) of miR-33a, miR-33b, and miR-33, respectively, that function as microRNAs in the cell.

"Antisense oligonucleotide" is an antisense nucleic acid (RNA or DNA) capable of hybridizing with a target nucleic acid. In the present application, antisense oligonucleotide means a molecule that binds to miRNA to inhibit the activity or function of the miRNA. The antisense oligonucleotides in the present application may include artificial nucleic acid molecules as well as naturally occurring nucleic acid molecules.

"siRNA (small interfering RNA)" is a small double-stranded RNA of 15 to 30 base pairs. siRNA is involved in the phenomenon called RNA interference, in which a target nucleic acid is destroyed to cause sequence-specific suppression of nucleic acid expression. In the present application, siRNA means a molecule that functions as an antagonist by destroying miR-33a or miR-33b to suppress the expression of miR-33a or miR-33b. The siRNAs in the present application may include artificial nucleic acid molecules as well as naturally occurring nucleic acid molecules.

"shRNA (short hairpin RNA)" has a hairpin structure, and undergoes processing to generate miRNA or siRNA (short interference RNA). In one embodiment, shRNA is transcribed from a shRNA expression vector in the cell.

"Complementary" means the capacity to cause pair formation between the nucleic acid bases of a first nucleic acid and a second nucleic acid. More specifically, for example, adenine is complementary to thymidine or uracil; cytosine is complementary to guanine; and 5-methylcytosine is complementary to guanine; but the above capacity is not limited to these.

"Fully complementary (also referred to as full complementarity)" or "100% complementary (also referred to as 100% complementarity)" means that every nucleic acid base in the nucleic acid base sequence of the first nucleic acid has its complementary nucleic acid base in the second nucleic acid base sequence of the second nucleic acid. In one aspect, the first nucleic acid is a modified oligonucleotide, and the second nucleic acid is a target nucleic acid.

"Hybridization" means annealing of a complementary nucleic acid molecule.

"Mismatch" or "non-complementary nucleic acid bases" means a case where a nucleic acid base of the first nucleic acid is incapable of forming a pair with the corresponding nucleic acid base of the second nucleic acid or the target nucleic acid.

"Prevention" means to delay or prevent development or occurrence of a disease, a disorder, or an undesirable health condition, or one or more symptoms associated with the disease, disorder, or undesirable health condition, for a period of several minutes to an indefinite length of time. The prevention also means to reduce the risk of occurrence of a disease, a disorder, or an undesirable health condition.

"Treatment" means to ameliorate, eliminate, or suppress the progression of a disease, a disorder, or an undesirable health condition, or one or more symptoms associated with the disease, disorder, or undesirable health condition, or to partially resolve or completely eliminate one or more causes of the disease, disorder, or undesirable health condition itself.

Embodiments of the present invention are described below in detail. The present invention is not limited to the following embodiments, and may be carried out with various modifications within the scope of the spirit of the invention.

### miR-33b Inhibitor

The present invention relates to a therapeutic or prophylactic agent for a myopathy, comprising a miR-33b inhibitor.

The miR-33b inhibitor comprises a substance that inhibits the expression or function of miR-33b.

Specific examples of the miR-33b inhibitor include substances that reduce the level of a molecule that functions as miR-33b in the cell (hereinafter referred to as "miR-33b-level-reducing substance"). The reduction of the miR-33b level means that the inhibitor has a function that acts on a direct target mRNA of miR-33b in the cell, to reduce the level(s) of the direct target mRNA and/or protein of miR-33b in the cell through destabilization or translation inhibition of the mRNA.

The reduction of the intracellular miR-33b level by the miR-33b-level-reducing substance means that, for example, when the miR-33b-level-reducing substance is brought into contact with a cell, the intracellular miR-33b level is reduced compared to a case where the substance is not brought into contact or where a negative control substance is brought into contact. Here, the degree of reduction of the miR-33b level in the cell may be any degree as long as the intracellular miR-33b level decreases, resulting in an increase(s) in the level(s) of a direct target mRNA of miR-33b and/or a direct target protein of miR-33b in the cell, or as long as the intracellular miR-33b level decreases, resulting in modification of the phenotype of the miR-33b-expressing cell to cause regulation of a disease-associated substance toward improvement of the disease. More specifically, for example, the degree of reduction is preferably one at which the contact of the miR-33-level-reducing substance with the cell results in reduction of the intracellular miR-33b level to not more than 50%, preferably not more than 30%, more preferably not more than 10% compared to a case where the substance is not brought into contact or where a negative control substance is brought into contact.

The miR-33b-level-reducing substance is not limited as long as it reduces at least the intracellular miR-33b level. The substance may also have an action that reduces the intracellular miR-33a level. In this case, the action of the substance to reduce the intracellular miR-33b level is preferably stronger than its action to reduce the intracellular miR-33a level. For example, in the later-described system in which the miR-33a and miR-33b levels are measured in animal anterior tibial muscle expressing miR-33a and miR-33b, a miR-33b-level-reducing substance whose miR-33b-level-reducing rate is higher than its miR-33a-level-reducing rate is preferably used. The miR-33b-level-reducing rate can be calculated, for example, as follows: 100 × (the miR-33b level in a case where no miR-33b-level-reducing substance is added - the miR-33b level in a case where a miR-33b-level-reducing substance is added) / the miR-33b level in a case where no miR-33b-level-reducing substance is added. The miR-33a-level-reducing rate can be calculated, for example, as follows: 100 × (the miR-33a level in a case where no miR-33b-level-reducing substance is added - the miR-33a level in a case where a miR-33b-level-reducing substance is added) / the miR-33a level in a case where no miR-33b-level-reducing substance is added.

Specific examples of the miR-33b-level-reducing substance whose miR-33b-level-reducing rate is higher than its miR-33a-level-reducing rate include miR-33b-level-reducing substances whose calculated value of the miR-33a-level-reducing rate / the miR-33b-level-reducing rate is not more than 0.8, not more than 0.7, not more than 0.6, not more than 0.5, not more than 0.4, not more than 0.3, not more than 0.2, or not more than 0.1, preferably not more than 0.6, more preferably not more than 0.3, still more preferably not more than 0.1.

The miR-33b inhibitor may be an inhibitor that inhibits the function of miR-33b. The inhibition of the function of miR-33b by the miR-33b inhibitor means inhibition of an inhibitory action of miR-33b on its direct target mRNA, and inhibition of a miR-33b-specific signaling pathway, which inhibition results in an increase in the expression level of the direct target mRNA and/or direct target protein of miR-33b, to cause regulation of a disease-associated substance toward improvement of the disease.

The miR-33b inhibitor preferably inhibits the expression or function of miR-33b, to result in an increase in the intracellular abundance level of a direct target mRNA of miR-33b and/or direct target protein of miR-33b.

Examples of the direct target mRNA of miR-33b include those to which miR-33b directly binds to reduce the expression level, such as ATP-binding cassette transporter A1 (ABCA1).

Direct target molecules of miR-33b, such as ABCA1, normally undergo inhibition of expression by miR-33b. Therefore, inhibition of miR-33b by the miR-33b inhibitor results in increases in their intracellular abundance levels.

The direct target mRNA may be any one or more of such direct target mRNAs.

In the present invention, the target mRNA of miR-33b includes mRNAs whose expression levels are directly or indirectly decreased by miR-33b. Examples of the target mRNA include Paired Box 7 (Pax7), Syntropin, Cycin Dependent Kinase 6 (Cdk6), CYCLIN D1 (CCND1), Follistatin (Fst), Utrophin, and Myogenietic Differentiation 1 (MyoD1, which may be hereinafter simply referred to as MyoD), whose intracellular expression levels are increased by miR-33b inhibitors.

Therefore, the miR-33b inhibitor preferably increases the expression level(s) of one or more of the mRNAs and/or proteins ABCA1, Pax7, Syntropin, Cdk6, CCND1, Follistatin, Utrophin, and MyoD in muscle cells such as myogenic cells, myoblasts, satellite cells, myotube cells, and multinucleated cells (which may be hereinafter simply referred to as "cells").

Enhancement of the expression level(s) of the mRNA and/or protein of ABCA1, Pax7, Syntropin, Cdk6, CCND1, Follistatin, Utrophin, MyoD, and the like; and promotion of differentiation (promotion of muscle differentiation) and maturation of myoblasts, growth of muscle fibers, and suppression of muscle fibrosis, based on such enhancement; have been reported to be factors important for improvement of muscular dystrophy (Non-Patent Document 5).

The promotion of muscle differentiation means promotion of differentiation from satellite cells into myoblasts or muscle cells.

The muscle fibers mean cells in a fibrous form formed by fusion of myotube cells. The muscle fibers gather to form muscle bundles, and the muscle bundles then gather to form a muscle tissue. The growth of muscle fibers can be evaluated using the later-mentioned increase in the muscle fiber cross-sectional area as an index.

The muscle fibrosis means accumulation of a substance such as collagen in the interstitium of a muscle tissue due to a muscle tissue disorder.

Regarding the genes of ABCA1, Pax7, Syntropin, Cdk6, CCND1, Follistatin, Utrophin, MyoD, and the like, enhancement of the expression level(s) of their mRNA and/or protein, and promotion of differentiation and maturation of myoblasts, growth of muscle fibers, and suppression of muscle fibrosis based on such enhancement, have been suggested to contribute to improvement of symptoms of diseases including not only muscular dystrophy, but also myopathies such as myasthenia gravis, inclusion body myositis, dermatomyositis, congenital myopathy, amyotrophic lateral sclerosis, and hereditary spastic paraplegia (Non-Patent Document 6). Thus, miR-33b inhibitors having an expression-enhancing action on these genes are expected to be prophylactic and therapeutic agents for the above diseases.

The degree to which the expression level of the target mRNA of miR-33b is increased by the miR-33b-level-reducing substance is not limited as long as a disease-associated substance regulated by the protein encoded by the target mRNA undergoes regulation toward improvement of the disease. Examples of such a degree of increase include those in cases where the contact of the miR-33b-level-reducing substance with the cell results in a not less than 1.2-fold, preferably not less than 1.5-fold, more preferably not less than 2-fold increase in the target mRNA expression level compared to cases where the substance is not brought into contact or a negative control substance is brought into contact with the cell.

The degree to which the expression level of the protein encoded by the target mRNA is increased by the miR-33b inhibitor is also not limited as long as a disease-associated substance regulated by the target protein undergoes regulation toward improvement of the disease. Examples of such a degree of increase include those in cases where the contact of the miR-33b-level-reducing substance with the cell results in a not less than 1.2-fold, preferably not less than 1.5-fold, more preferably not less than 2-fold increase in the target protein expression level compared to cases where the substance is not brought into contact or a negative control substance is brought into contact with the cell.

The degree to which the expression level(s) of the mRNA(s) of one or more selected from ABCA1, Pax7, Syntropin, Cdk6, CCND1, Follistatin, Utrophin, and MyoD or the expression level(s) of the protein(s) encoded thereby is/are increased by the miR-33b-level-reducing substance is not limited as long as a disease-associated substance is regulated toward improvement of the disease. Examples of such a degree of increase include those in cases where administration of the miR-33b-level-reducing substance to a myopathy model animal (such as an mdx mouse) results in a not less than 1.2-fold, not less than 1.3-fold, not less than 1.4-fold, not less than 1.5-fold, not less than 1.6-fold, not less than 1.7-fold, not less than 1.8-fold, not less than 1.9-fold, or not less than 2.0-fold, preferably not less than 1.5-fold, more preferably not less than 1.8-fold, still more preferably not less than 2.0-fold increase in the expression level(s) of the mRNA(s) of one or more selected from ABCA1, Pax7, Syntropin, Cdk6, CCND1, Follistatin, Utrophin, and MyoD or the expression level(s) of the protein(s) encoded thereby, compared to cases where the substance is not administered or a negative control substance is administered.

Specific examples of the regulation toward improvement of the disease include regulations toward a condition in which promotion of muscle differentiation, growth of muscle fibers (for example, an increase in the muscle fiber cross-sectional area), and/or suppression of muscle fibrosis is/are found.

The degree to which the muscle fiber cross-sectional area is increased by the miR-33b-level-reducing substance is not limited as long as administration of the miR-33b-level-reducing substance to a myopathy model animal (such as an mdx mouse) results in an increase in the muscle fiber cross-sectional area compared to cases where the substance is not administered or a negative control substance is administered. Examples of such a degree of increase include those in cases where the ratio of muscle fibers having a muscle fiber cross-sectional area of 300 to 500 (µm²) increases not less than 1.3-fold, not less than 1.4-fold, not less than 1.5-fold, not less than 1.6-fold, not less than 1.7-fold, not less than 1.8-fold, not less than 1.9-fold, not less than 2.0-fold, not less than 2.5-fold, or not less than 3.0-fold, preferably not less than 1.5-fold, more preferably not less than 2.0-fold, still more preferably not less than 3.0-fold compared to cases where the substance is not administered or a negative control substance is administered.

The degree to which muscle fibrosis is suppressed by the miR-33b-level-reducing substance is not limited as long as administration of the miR-33b-level-reducing substance to a myopathy model animal (such as an mdx mouse) results in a decrease in muscle fibrosis compared to cases where the substance is not administered or a negative control substance is administered. Examples of such a degree of suppression include a not more than 0.8-fold, not more than 0.7-fold, not more than 0.6-fold, not more than 0.5-fold, not more than 0.4-fold, not more than 0.3-fold, not more than 0.2-fold, or not more than 0.1-fold, preferably not more than 0.6-fold, more preferably not more than 0.3-fold, still more preferably not more than 0.1-fold decrease in muscle fibrosis.

A miR-33b inhibitor can be used for promotion of muscle differentiation, growth of muscle fibers, and suppression of muscle fibrosis.

Specific examples of the miR-33b-level-reducing substance include antisense oligonucleotides, siRNAs, and shRNAs against miR-33b, and vectors that express these. An antisense oligonucleotide against miR-33b is preferably used. As a miR-33b inhibitor, a compound that inhibits the function of miR-33b may also be used.

The antisense oligonucleotide against miR-33b is an antisense oligonucleotide that reduces the intracellular miR-33b level, which antisense oligonucleotide 1) is composed of 7 to 20 residues, and 2) has a base sequence which is not less than 90% complementary to an equal length portion of the miR-33b stem-loop sequence of SEQ ID NO:4, preferably of the miR-33b nucleic acid base sequence of SEQ ID NO: 1 (also referred to as hsa-miR-33b-5p). The equal length portion means a portion in a specific base sequence where the nucleic acid base sequence of each antisense oligonucleotide and the nucleic acid base sequence of miR-33b are complementary to each other. More specifically, although the nucleic acid base sequence of the antisense oligonucleotide against miR-33b is preferably fully complementary to the equal length portion of the nucleic acid base sequence of miR-33b, the nucleic acid base sequence of the antisense oligonucleotide may contain one or more mismatched nucleic acid bases. Thus, an antisense oligonucleotide with a complementarity of not less than 85%, not less than 90%, preferably not less than 95% is used. The nucleic acid base sequence of the antisense oligonucleotide against miR-33b having such a mismatch is not limited as long as the antisense oligonucleotide is capable of hybridizing with miR-33b to decrease the intracellular miR-33b level. For reducing binding to miR-33a, no mismatch is preferably present at either the 9th or 10th nucleic acid base as counted from the 5'-end of the miR-33b nucleic acid base sequence of SEQ ID NO: 1.

The above mismatched nucleic acid bases may be clustered, may be sandwiching a complementary nucleic acid base(s), or may be non-consecutive to each other or to complementary nucleic acid bases. The complementarity percentage of the antisense oligonucleotide against miR-33b to miR-33b can be conventionally determined using, for example, the BLAST program (basic local alignment search tools), the PowerBLAST program (Altschul et al., J. Mol. Biol., 1990, 215, 403 410; Zhang and Madden, Genome Res., 1997, 7, 649 656), or Genetyx software (GENETYX CORPORATION), which is known in the art. The complementarity can be determined by a gap program (Wisconsin Sequence Analysis Package, Version 8 for Unix, Genetics Computer Group, University Research Park, Madison Wis.) with the default setting using the algorithm of Smith and Waterman (Adv. Appl. Math., 1981, 2, 482 489) and the Genetyx software (GENETYX CORPORATION).

The miR-33b nucleic acid base sequence of SEQ ID NO: 1 is included in the miR-33b stem-loop sequence, and examples of the miR-33b nucleic acid base sequence to be targeted by the antisense oligonucleotide against miR-33b includes the full-length miR-33b stem-loop sequence.

The nucleic acid base sequence of the antisense oligonucleotide against miR-33b may be any equal length portion of the complementary strand of the base sequence of the miR-33b stem-loop sequence of SEQ ID NO:4, preferably of the complementary strand of the miR-33b base sequence of SEQ ID NO: 1. The sequence length may be any length, and examples of the sequence length include 7, 8, 9, 10, 11, 12, 13, 14, 15, 16, 17, 18, 19, and 20 residues. The nucleic acid base sequence of the antisense oligonucleotide preferably includes the complementary sequence of the 9th to 10th residues of the miR-33b nucleic acid base sequence of SEQ ID NO:1 as counted from the 5'-end, and has a sequence length of preferably 12 to 19 residues, more preferably 12 to 16 residues. Specific examples of the sequence include
AACAGCAATGCA (SEQ ID NO:5) and
TGCAACAGCAATGCAC (SEQ ID NO:6).
AACAGCAATGCA (SEQ ID NO:5)
is preferably used.

In one aspect, in the antisense oligonucleotide, part or all of the cytosine bases in the sequence of SEQ ID NO:5 or 6 are 5-methylcytosine. Examples of the modified oligonucleotide in which part of the cytosine bases is 5-methylcytosine include SEQ ID NO:8.

The antisense oligonucleotide against miR-33b may be a modified oligonucleotide. The modified oligonucleotide may contain a modified base such as 5-methylcytosine as described above, or may be an oligonucleotide in which at least one nucleoside constituting it contains a modified sugar.

The modified sugar means a modified sugar moiety. A modified oligonucleotide containing one or more modified sugars has advantageous properties such as enhanced nuclease stability and increased binding affinity. As at least one of the modified sugars, a bicyclic sugar or a substituted sugar moiety is preferably included.

Examples of the nucleosides containing a modified sugar include nucleosides containing 5'-vinyl, 5'-methyl (R or S), 4'-S, 2'-F, 2'-OCH₃, 2'-OCH₂CH₃, 2'-OCH₂CH₂F, or 2'-O(CH₂ )₂OCH₃ as a substituent. The substituent at the 2'-position may also be selected from allyl, amino, azide, thio, O-allyl, O-C₁-C₁₀ alkyl, OCF₃, OCH₂F, O(CH₂)₂SCH₃, O(CH₂)₂-O-N(Rₘ)(Rₙ), O-CH₂-C(=O)-N(Rₘ)(Rₙ), and O-CH₂-C(=O)-N(R₁)-(CH₂)₂-N(Rₘ)(Rₙ) (wherein Rₗ, Rₘ, and Rₙ are each independently H or a substituted or unsubstituted C₁-C₁₀ alkyl).

Examples of the nucleosides containing a bicyclic sugar include nucleosides containing a cross-link between the 4'- and 2'-ribosyl ring atoms. In one aspect, the oligonucleotide provided in the present description includes nucleosides containing one or more bicyclic sugars including a cross-link of one of the following formulae: 4'-(CH₂)-O-2' (LNA); 4'-(CH₂)-S-2'; 4'-(CH₂)₂-O-2' (ENA); 4'-CH(CH₃)-O-2'; and 4'-CH(CH₂OCH₃)-O-2' (and analogs thereof; see US 7399845 B); 4'-C(CH₃)(CH₃)-O-2' (and analogs thereof; see WO2009/006478); 4'-CH₂-N(OCH₃)-2' (and analogs thereof; see WO2008/150729); 4'-CH₂-O-N(CH₃)-2' (see US 2004-0171570 A); 4'-CH₂-N(R)-O-2' (wherein R is H, C₁-C₁₂ alkyl, or a protective group) (see US 7427672 B); 4'-CH₂-C(H)(CH₃)-2' (see Chattopadhyaya et al., J. Org. Chem., 2009, 74, 118-134); and 4'-CH₂-C(=CH₂)-2' (and analogs thereof; see WO2008/154401).

In one aspect (AmNA), examples of the nucleosides containing a bicyclic sugar include nucleosides of the following formula: 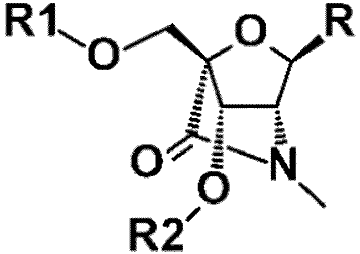 AmNA
[wherein R is a nucleic acid base, and R1 and R2 are each independently a phosphate group that is optionally substituted].
Methods of preparing the modified sugars are well known to those skilled in the art as described in, for example, WO11/052436.

In one aspect, the antisense oligonucleotide against miR-33b has a nucleic acid base sequence in which at least one nucleic acid base is cytosine. In one aspect, at least one cytosine is 5-methylcytosine, which is a modified nucleic acid base. In one aspect, every cytosine is 5-methylcytosine.

The naturally occurring internucleoside bonds in RNA and DNA are 3'-5' phosphodiester bonds. Oligonucleotides containing one or more modified, that is, non-naturally occurring, internucleoside bonds are often more preferred than oligonucleotides with naturally occurring internucleoside bonds, because of properties such as enhancement of the cellular uptake, enhancement of the affinity to the target nucleic acid, and enhancement of the stability in the presence of nuclease.

Examples of the modified internucleoside bonds contained in the oligonucleotides include internucleoside bonds containing a phosphorus atom and internucleoside bonds containing no phosphorus atom. Representative examples of the phosphorus-containing internucleoside bonds include, but are not limited to, one or more of phosphodiester, phosphotriester, methylphosphonate, phosphoramidate, and phosphorothioate. Methods of preparing the phosphorus-containing or non-phosphorus-containing bonds are well known.

In one aspect, all internucleoside bonds of the modified oligonucleotide against miR-33b are phosphorothioate internucleoside bonds.

In each of the symbols representing nucleotides, such as "Gas", in the present description, the abbreviation shown in the left position refers to the nucleic acid base moiety; the abbreviation shown in the middle position refers to the sugar moiety, and the abbreviation shown in the right position refers to the mode of the internucleoside bond.

In one aspect, the antisense oligonucleotide is a modified oligonucleotide of the following formula:
Aas Ads Mas Ads Gas Cds Aas Ads Tas Gds Mas Ad (SEQ ID NO:8) wherein in the formula,
each nucleic acid base is represented according to the following symbol: A = adenine, T = thymine, G = guanine, or C = cytosine;
each sugar moiety is represented according to the following symbol: a = the sugar moiety of AmNA, or d = 2'-deoxyribose; and
each internucleoside bond is represented according to the following symbol: s = phosphorothioate.

### Method of Selecting miR-33b Inhibitor

The method of selecting the miR-33b inhibitor is not limited as long as the method enables testing of reduction of the intracellular miR-33b level or testing of inhibition of the function due to the miR-33b inhibitor. Since a miR-33b-level-reducing substance whose miR-33b-level-reducing rate is higher than its miR-33a-level-reducing rate is preferably used as the miR-33b inhibitor, a method that enables testing of reduction of the intracellular levels of miR-33b and miR-33a is preferably used. Specific examples of the method include the following in vitro and in vivo test methods.

The in vitro test of the reduction of the intracellular miR-33b level due to the miR-33b inhibitor may be any test as long as the test is based on a cell system in which miR33b is expressed, preferably a cell system in which miR-33b and miR-33a are expressed (which may be hereinafter referred to as "miR-33b expression cell system"). Examples of the cell system include muscle cells collected from mice expressing miR-33a and miR-33b. A cell system in which human miR-33b is introduced, preferably human miR-33b and miR-33a are introduced, may also be selected. The cell system used for the introduction of miR-33b, or miR-33b and miR-33a, is not limited as long as the cell system is an animal-derived cell that is normally used. These cell systems are available from commercial suppliers, and cultured in accordance with the supplier's instructions using commonly used, commercially available reagents. Examples of the method of introducing miR-33b into these cell systems include, but are not limited to, a method in which transfection is carried out with a vector for expression of miR-33b, or miR-33b and miR-33a.

The method of bringing the miR-33b inhibitor into contact with the miR-33b expression cell system is also not limited. Examples of the method include a method commonly used for introducing nucleic acid into cells. Specific examples of the method include lipofection, electroporation, and gymnosis.

The intracellular level of miR-33b or miR-33a can be assayed using a variety of methods known in the art. Specific examples of such methods include Northern blot analysis, competitive polymerase chain reaction (PCR), and quantitative real-time PCR. Isolation of miRNAs are possible by using a method well known in the art, for example, by using the TriPure Isolation Reagent (Roche), Maxwell RSC miRNA Tissue Kit reagent (Promega), or the like in accordance with the manufacturer's recommended protocols. The expression level(s) of miR-33b, or miR-33b and miR-33a, can thus be analyzed.

An in vivo test may be carried out in order to evaluate the fact that the miR-33b inhibitor reduces the intracellular miR-33b level, or to evaluate the ability of the miR-33b inhibitor to change the phenotype of a miR-33b-expressing cell. Examples of the test of the miR-33b inhibitor regarding reduction of the intracellular miR-33b level include a method in which the miR-33b inhibitor is administered to an animal expressing miR33b, and then, for example, the miR-33b level, or the miR-33b and miR-33a levels, in the cells of anterior tibial muscle is analyzed. The evaluation of the ability of the miR-33b inhibitor to change the phenotype of miR-33b-expressing cells can be carried out using an experimental disease model such as a muscular dystrophy model, for example, an mdx mouse model.

An in vitro or in vivo test of the miR-33b inhibitor regarding reduction of the miR-33b level or inhibition of the function of miR-33b can be carried out by administering the miR-33b inhibitor into a miR-33b-expressing cell system or an animal expressing miR-33b, preferably miR-33b and miR-33a, and then measuring the expression level(s) of one or more of the mRNAs and/or proteins selected from ABCA1, Pax7, syntropin, Cdk6, CCND1, Follistatin, Utrophin, and MyoD.

The miR-33b inhibitor obtained by the selection method of the present invention can be used as a prophylactic and/or therapeutic agent for myopathies since the inhibitor suppresses the occurrence or progression of myopathies. Thus, the present invention provides a method of screening for a prophylactic and/or therapeutic agent for a myopathy, the method comprising a step of selecting a miR-33b inhibitor by the above method.

### Method of Producing Antisense Oligonucleotide against miR-33b

The antisense oligonucleotide against miR-33b can be synthesized by an ordinary method. For example, it can be easily synthesized by a commercially available nucleic acid synthesizer. The nucleoside with sugar modification AmNA, which may be contained in the antisense oligonucleotide, can be synthesized by the method disclosed in WO11/052436.

siRNA and shRNA against miR-33b can be obtained by artificial, chemical synthesis. The siRNA and shRNA can be synthesized, for example, by in vitro synthesis of antisense and sense RNAs from a template DNA using T7 RNA polymerase and T7 promoter.

Compounds having an activity that inhibits miR-33b can be synthesized by, for example, the methods disclosed in Proc. Natl. Acad. Sci. USA. 113(21): 5898-903 (2016), Mol. Pharm. 16(2): 914-920 (2019), and J. Am. Chem. Soc. 139(9): 3446-3455 (2017).

### Treatment of Myopathy Using miR-33b Inhibitor

Using a miR-33b inhibitor, a myopathy can be treated or prevented.

As a result of study by the present inventors, a miR-33b inhibitor reduced the intracellular miR-33b level, and improved symptoms of a myopathy through promotion of muscle differentiation, growth of muscle fibers, and suppression of muscle fibrosis, thereby suppressing the progression of the myopathy as shown below in the Examples.

In the present invention, the growth of muscle fibers includes an increase in thick muscle fibers and a decrease in thin muscle fibers.

Examples of the mechanism of improving a myopathy by the miR-33b inhibitor include, as shown below in the Examples, enhancement of the expression of a target mRNA and/or protein such as ABCA1, Pax7, syntropin, Cdk6, CCND1, Follistatin, Utrophin, and MyoD. However, since there are a number of downstream targets of miR-33b and a plurality of downstream signaling pathways, the mechanism is not limited thereto. In contrast, an antisense oligonucleotide against miR-33a did not cause promotion of muscle differentiation, growth of muscle fibers, or suppression of muscle fibrosis.

Thus, the present invention clarified for the first time the role of miR-33b in a myopathy, and led to the discovery that use of an inhibitor which selectively inhibits miR-33b enables prevention or treatment of the myopathy.

Thus, a miR-33b inhibitor is useful as a therapeutic or prophylactic agent for a myopathy since it is capable of suppressing the occurrence or progression of the myopathy, capable of improving the symptoms, and/or capable of maintaining or improving the motor function through enhancement of the muscle strength (including suppression of a decrease in the muscle strength), to produce a prophylactic or therapeutic effect.

The myopathy is not limited as long as it is a disease in muscle, and as long as the disease can be prevented or treated through promotion of muscle differentiation, growth of muscle fibers, and/or suppression of muscle fibrosis. Examples of the myopathy include neurogenic muscle atrophy, myogenic muscle atrophy, and diseases of the neuromuscular junction, preferably include myogenic muscle atrophy, more preferably include muscular dystrophy.

Examples of the neurogenic muscle atrophy include spinal muscular atrophy, spinal and bulbar muscular atrophy, and amyotrophic lateral sclerosis.

Examples of the myogenic muscle atrophy include muscular dystrophy, congenital myopathy, inflammatory myopathy, metabolic myopathy, and muscle injuries due to endocrine diseases. Here, specific examples of the muscular dystrophy include congenital muscular dystrophy, limb-girdle muscular dystrophy, facioscapulohumeral muscular dystrophy, and myotonic dystrophy. Specific examples of the inflammatory myopathy include polymyositis, dermatomyositis, and inclusion body myositis. Specific examples of the metabolic myopathy include myopathies due to inborn errors of metabolism, and periodic paralysis. Specific examples of the muscle injuries due to endocrine diseases include muscle injuries due to, for example, thyroid diseases and diabetes.

Examples of the diseases of the neuromuscular junction include myasthenia gravis, myasthenic syndrome, and congenital myasthenia.

Examples of the other myopathies include diseases and symptoms that cause decreases in the muscle function, such as cachexia, sarcopenia, disuse atrophy, and hereditary spastic paraplegia.

As described above, a miR-33b inhibitor can be used for treating or preventing a myopathy. Therefore, the invention provides a miR-33b inhibitor for use in the treatment of a myopathy; a pharmaceutical composition for use in the treatment of a myopathy; use of a miR-33b inhibitor for the treatment of a myopathy; use of a miR-33b inhibitor for the production of a therapeutic drug for a myopathy; a miR-33b inhibitor for use in the production of a therapeutic drug for a myopathy; and a therapeutic or prophylactic method for a myopathy, the method comprising administering an effective amount of a miR-33b inhibitor to a subject in need thereof.

A composition comprising: a miR-33b inhibitor or a pharmaceutically acceptable salt thereof; and a pharmaceutically acceptable carrier; can be used as a pharmaceutical composition. For the preparation of the pharmaceutical composition, an antisense oligonucleotide may be mixed with one or more pharmaceutically acceptable, active or inactive substances. The composition and the method for the formulation of the pharmaceutical composition may be selected based on several criteria including the administration route, severity of the disease, and the administration dose.

For example, as a composition for parenteral administration, an injection solution or the like is used. Examples of the dosage form of the injection solution include an intravenous injection solution, a subcutaneous injection solution, an intradermal injection solution, an intramuscular injection solution, an infusion solution, and an intra-articular injection solution. The composition is preferably an intravenous injection solution or intramuscular injection solution. The injection solution is prepared according to a per se known method, for example, by dissolving, suspending, or emulsifying the antisense oligonucleotide in a sterile aqueous or oily liquid that is commonly used for injection solutions. Examples of the aqueous liquid used for the injection include phosphate-buffered saline, physiological saline, and isotonic solutions containing glucose and other auxiliary agents. The aqueous liquid may be used in combination with an appropriate solubilizer such as an alcohol (for example, ethanol), a polyalcohol (for example, propylene glycol or polyethylene glycol), a nonionic surfactant [for example, polysorbate 80 or HCO-50 (polyoxyethylene (50 mo1) adduct of hydrogenated castor oil)]. The aqueous liquid may also contain a buffer, a pH adjusting agent, an isotonic agent, a soothing agent, a preservative, a stabilizer or the like.

Examples of the composition for oral administration include those in a solid or liquid dosage form, more specifically, tablets (including sugar-coated tablets and film-coated tablets), pills, granules, powders, capsules (including soft capsules), syrups, emulsions, and suspensions. Such a composition is produced by a per se known method, and contains a carrier, diluent, or excipient commonly used in the field of pharmaceutical preparations. Examples of the carrier or excipient used for the tablets include lactose, starch, sucrose, and magnesium stearate.

The pharmaceutical composition of the present invention may contain a reagent for introduction of nucleic acid. Examples of the reagent used for introduction of nucleic acid include cationic lipids such as liposomes, lipofectin, lipofectamine, DOGS (transfectam), DOPE, DOTAP, DDAB, DHDEAB, HDEAB, polybrene, and poly(ethyleneimine) (PEI).

As the antisense oligonucleotide contained in the pharmaceutical composition of the present invention, an antisense oligonucleotide conjugated, at each of one or more sites, with a conjugate group such as cholesterol, a carbohydrate, a phospholipid, biotin, phenazine, a vitamin, a peptide, folate, phenanthridine, anthraquinone, acridine, fluorescein, rhodamine, coumarin, or a pigment is preferably used. The conjugated antisense oligonucleotide may be selected from the viewpoint of its activity, and enhancement of incorporation into a tissue or cell for targeting the affected area of myopathy.

The conjugate group is preferably cholesterol or a lipid.

The conjugate group is directly bound to the antisense oligonucleotide, or the conjugate group is bound to the antisense oligonucleotide through a linking moiety selected from amino, hydroxyl, carboxylate, thiol, an unsaturated moiety (such as a double or triple bond), 8-amino-3,6-dioxaoctanoic acid (ADO), succinimidyl 4-(N-maleimidomethyl)cyclohexane-1-carboxylate (SMCC), 6-aminohexanoic acid (AHEX or AHA), substituted C₁-C₁₀ alkyl, substituted or unsubstituted C₂-C₁₀ alkenyl, and substituted or unsubstituted C₂-C₁₀ alkynyl. Here, the substituent is selected from amino, alkoxy, carboxy, benzyl, phenyl, nitro, thiol, thioalkoxy, halogen, alkyl, aryl, alkenyl, and alkynyl.

The pharmaceutical composition of the present invention may be selected from the viewpoint of reducing the side effects. Examples of the side effects include injection site reaction, abnormality in a liver function test, renal function abnormality, hepatotoxicity, nephrotoxicity, central nervous system abnormality, myopathy, and malaise. For example, an elevated blood level of ALT, AST, or γ-GTP may indicate hepatotoxicity or liver function abnormality. For example, an elevated bilirubin level may indicate hepatotoxicity or liver function abnormality. An elevated urine protein level, or an elevated blood level of creatinine or BUN may indicate nephrotoxicity or renal function abnormality.

Treatment, prevention, or amelioration of a myopathy is possible by administration of the pharmaceutical composition of the present invention to a subject individual by an appropriate method. Thus, the present invention provides a method of treatment, prevention, or amelioration of a myopathy, the method comprising administering an effective amount of a miR-33b inhibitor, preferably an antisense oligonucleotide against miR-33b, or a pharmaceutical composition containing it, to a subject individual in need thereof by an appropriate method.

The dosage form of the pharmaceutical composition of the present invention may be ordinary systemic administration such as intravenous or intraarterial administration, or may be local administration such as local injection or oral administration. Administration by intravenous injection or subcutaneous injection is preferred. The dose of the pharmaceutical composition of the present invention may be appropriately adjusted depending on the purpose of use, the severity of the disease, the age, body weight, and gender of the patient, and the like. Usually, the dose may be selected within the range of 0.1 ng to 100 mg/kg/day, preferably 1 ng to 10 mg/kg/day, in terms of the amount of the antisense oligonucleotide.

### EXAMPLES

### Unlimited Disclosure, and Incorporation by Reference

Although certain compounds, compositions, and methods described herein are described specifically in accordance with certain aspects, the following Examples merely serve to illustrate the compounds described herein, and do not intend to limit such compounds. Each reference described in the present application is incorporated herein by reference in its entirety.

### Example 1

### Synthesis and Purification of Antisense Oligonucleotides

AmNA amidite was obtained from Osaka Synthetic Chemical Laboratories, Inc., and AmNA-containing antisense oligonucleotides were synthesized and purified by Ajinomoto Bio-Pharma Services - GeneDesign, Inc.

The synthesized antisense oligonucleotides are shown in Table 1. 33b-2-AmNAis an oligonucleotide against miR-33b (which may be hereinafter referred to as "anti-miR-33b"); 33a-2-AmNAis an oligonucleotide against miR-33a (which may be hereinafter referred to as "anti-miR-33a"); and NEG-AmNA is a control antisense oligonucleotide (which may be hereinafter referred to as "anti-miR-ctl"). Each nucleotide in each antisense oligonucleotide is represented by three letters. However, the nucleotide at the 3'-end is represented by two letters because of the absence of the internucleoside bond.
1) The first letter is represented by an upper-case letter, and denotes the following nucleic acid bases:
   A = adenine, T = thymine, G = guanine, C = cytosine, M = 5-methylcytosine.
2) The second letter denotes the following sugar moieties:
   a = the sugar moiety of AmNA, d = 2'-deoxyribose.
3) The third letter denotes the following internucleoside bond:
   s = phosphorothioate.

**Table 1**

| Antisense oligonucleotide name | SEQ ID NO: | Sequence | m/z |
|---|---|---|---|
| 33a-2-AmNA(12) | 7 | AasAdsMasTdsAasCdsAasAdsTasGdsMasAd | 4156.5 |
| 33b-2-AmNA(12) | 8 | AasAdsMasAdsGasCdsAasAdsTasGdsMasAd | 4181.33 |
| NEG-AmNA (12) | 11 | AasAdsMasAdsAasTdsAasCdsTasAdsMasGd | 4156.15 |

### Example 2

### Measurement of Muscle-Formation-Promoting Action of miR-33b Antisense Oligonucleotide in Primary Skeletal Muscle Cells Derived from miR-33b Knock-in/mdx Mice

miR-33b knock-in mice produced using C57BL/6 as the background were mated with dystrophin-deficient mdx mice, to obtain miR-33b knock-in/mdx mice. miR-33b is absent in mice, but present in humans. Therefore, for the purpose of investigating the effect of a miR-33b antisense oligonucleotide, the miR-33b knock-in was carried out to force expression of miR-33b in the myopathy model mice.

Primary skeletal muscle cells were established from 8-week-old miR-33b knock-in/mdx mice by the following method. In the method, skeletal muscle (anterior tibial muscle) was removed, and muscle fibers were isolated with 0.2% collagenase Type 2 solution, followed by performing primary culture on a thin silicone membrane coated with collagen type 1. The cultured cells were myoblasts (progenitor cells). After the growth, the medium was replaced with 2% horse serum medium to allow differentiation of the myoblasts into muscle cells, and the cells were then allowed to fuse, to form myotube cells.

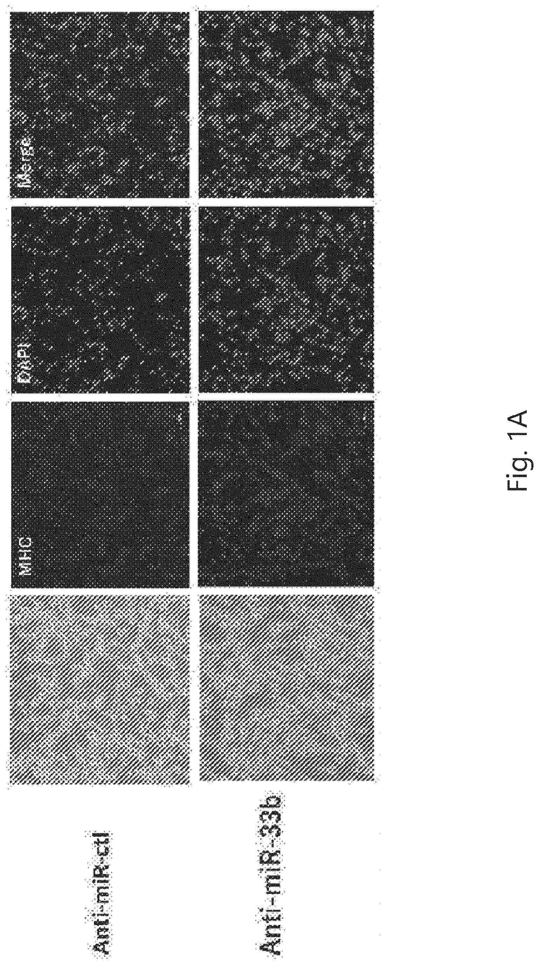

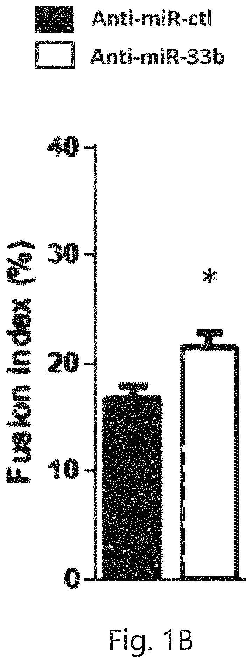

anti-miR-ctl or anti-miR-33b were added at 25 nM to the primary skeletal muscle cells, and the muscle formation-promoting action on Day 3 was measured based on detection of myosin heavy chain (MHC) and the myotube cell fusion rate (Figs. 1A and 1B). As a result, the miR-33b antisense oligonucleotide showed a significant muscle formation-promoting action relative to the control.

### Measurement of Various Parameters of Skeletal Muscle after Single Administration of Antisense Oligonucleotide to Skeletal Muscle in miR-33b Knock-in/mdx Mice

To 8-week-old miR-33b knock-in/mdx mice, 50 µl (10 mg/ml) of an antisense oligonucleotide (anti-miR-ctl, anti-miR-33b, or anti-miR-33a) was administered at both anterior tibial muscles, and anterior tibial muscle at the administration sites was sampled three days thereafter, followed by measuring the expression of miR-33a and miR-33b by the following method. RNA was separated and purified using the TriPure Isolation Reagent (Roche), and the miRNAs were measured using the TaqMan MicroRNA assay protocols (Applied Biosystems). The expression levels were analyzed using a thermal cycler (ABI Prism Step One Plus sequence detection system), and standardized using U6 snRNA.

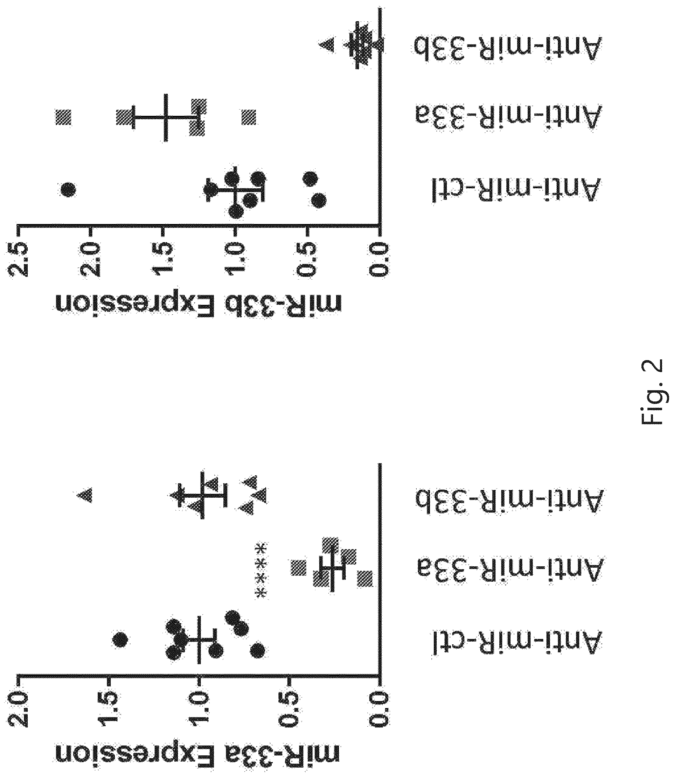

As a result (Fig. 2), the miR-33b antisense oligonucleotide was found to have hardly changed the expression of miR-33a, but strongly reduced the expression of miR-33b. The miR-33a antisense oligonucleotide hardly changed the expression of miR-33b, but strongly reduced the expression of miR-33a. The value of the miR-33a-level-reducing rate / miR-33b-level-reducing rate obtained by the miR-33b antisense oligonucleotide was 0.02.

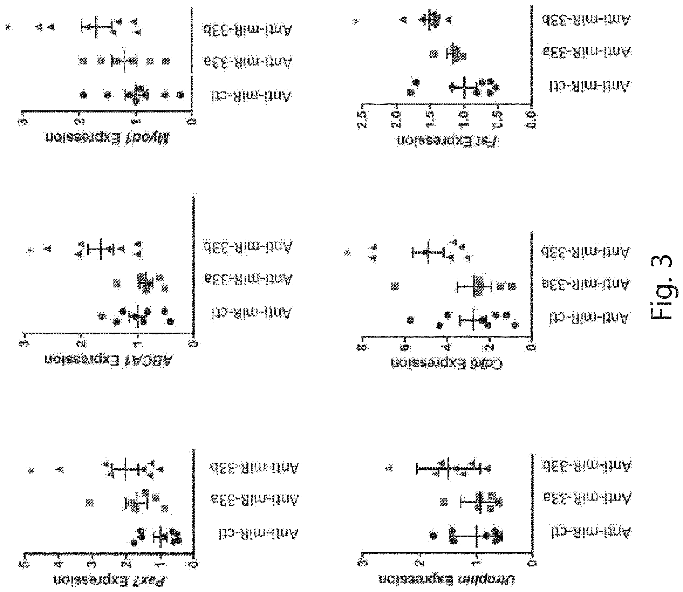

To 8-week-old miR-33b knock-in/mdx mice, 50 µl (10 mg/ml) of an antisense oligonucleotide (anti-miR-ctl, anti-miR-33b, or anti-miR-33a) was administered at both anterior tibial muscles, and anterior tibial muscle at the administration sites was sampled three days thereafter, followed by analyzing the expression of various genes. As a result, as shown in Fig. 3, the miR-33b antisense oligonucleotide was found to have caused 1.65-fold, 1.76-fold, 1.70-fold, 1.49-fold, 1.50-fold, and 2.03-fold increases in the expression of ABCA1, Cdk6, MyoD, Utrophin, Fst, and Pax7, respectively, relative to the control antisense oligonucleotide. On the other hand, the miR-33a antisense oligonucleotide did not increase the expression of these genes.

The RNA extraction and the real-time PCR were performed by the following procedure. RNA was isolated and purified using the TriPure Isolation Reagent (Roche), and cDNA was synthesized using the Verso cDNA Synthesis Kit (Thermo Scientific) in accordance with the manufacturer's instructions, followed by 40 cycles of amplification of specific genes using the THUNDERBIRD PCR Master Mix (TOYOBO). The expression levels were standardized using GAPDH as a housekeeping gene. The sequences of the primers used were as follows.
ABCA1 Forward, AACAGTTTGTGGCCCTTTTG (SEQ ID NO:9)
ABCA1 Reverse, AGTTCCAGGCTGGGGTACTT (SEQ ID NO:10)
Cdk6 Forward, TGTTTCAGCTTCTCCGAGGT (SEQ ID NO: 12)
Cdk6 Reverse, CTGGACTGGAGCAGGACTTTC (SEQ ID NO: 13)
MyoD Forward, CTTCTACGCACCTGGACCG (SEQ ID NO:14)
MyoD Reverse, ACTGTAGTAGGCGGTGTCGT (SEQ ID NO: 15)
Utropin Forward, GCCCTCCCTGCAGATTATTTGG (SEQ ID NO: 16)
UtrophinTROPHIN Reverse, CTGTCCAGTTGACCTTTGATACTCTTC (SEQ ID NO:17)
Pax7 Forward, GAGTTCGATTAGCCGAGTGC (SEQ ID NO: 18)
Pax7 Reverse, GTCGGGTTCTGATTCCACAT (SEQ ID NO: 19)
Fst Forward, ATGGACCGAGGAGGATGTGA (SEQ ID NO:20)
Fst Reverse, TTGCATCTGGCCTTGAGGAG (SEQ ID NO:21)
GAPDH Forward, AAATGGTGAAGGTCGGTGTG (SEQ ID NO:22)
GAPDH Reverse, AATCTCCACTTTGCCACTGC (SEQ ID NO:23)

### Example 3

### Measurement of Various Parameters of Skeletal Muscle after Repeated Administration of Antisense Oligonucleotide to Skeletal Muscle in miR-33b Knock-in/mdx Mice

To 8-week-old miR-33b knock-in/mdx mice, 50 µl (1 mg/ml) of an antisense oligonucleotide (anti-miR-ctl, anti-miR-33b, or anti-miR-33a) was administered at both anterior tibial muscles, and the antisense oligonucleotide was similarly administered 7 days, 14 days, 21 days, and 35 days thereafter. Three days after the final administration, anterior tibial muscle at the administration sites was sampled, and subjected to various analyses.

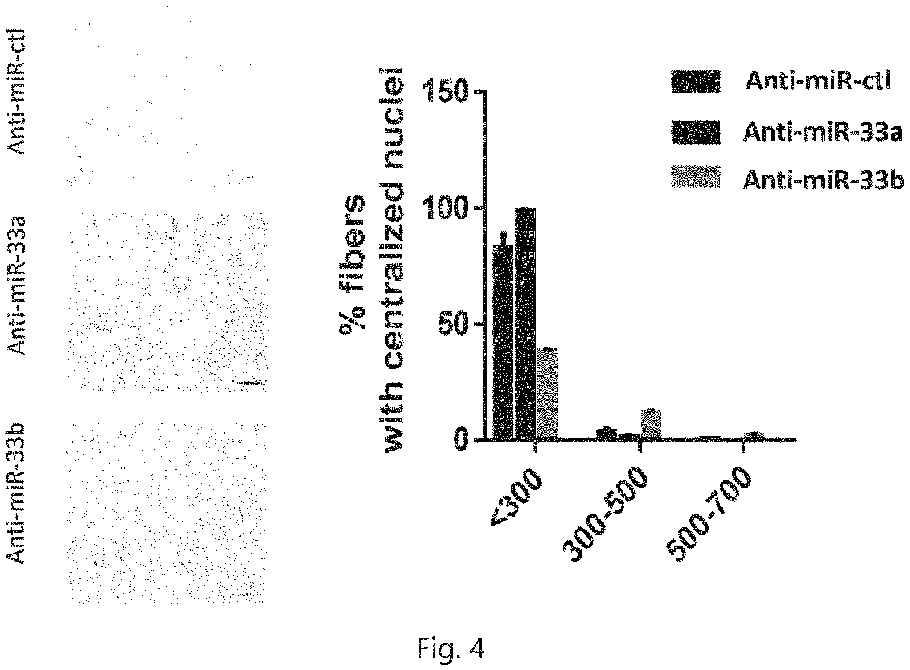

A change in the size of the muscle cross-sectional area of skeletal muscle fibers in the anterior tibial muscle of the miR-33b knock-in /mdx mice caused by the administration of the miR-33b antisense oligonucleotide was quantitatively analyzed using Imaged (National Institute of Health). As a result, as shown in Fig. 4, the miR-33b antisense oligonucleotide was found to have caused a significant, 3.2-fold increase in the ratio of muscle fibers having a cross-sectional area of 300 to 500 (µm²) relative to the control. Further, the miR-33b antisense oligonucleotide reduced the ratio of thin muscle fibers corresponding to fibers having a cross-sectional area of not more than 300 (µm²), and increased the ratio of muscle fibers having a cross-sectional area of 500 to 700 (µm²). On the other hand, the miR-33a antisense oligonucleotide increased the ratio of fibers having a cross-sectional area of not more than 300 (µm²), and reduced the ratios of muscle fibers having a cross-sectional area of 300 to 500 (µm²) or 500 to 700 (µm²). Thus, the miR-33b antisense oligonucleotide was found to cause the growth of muscle fibers.

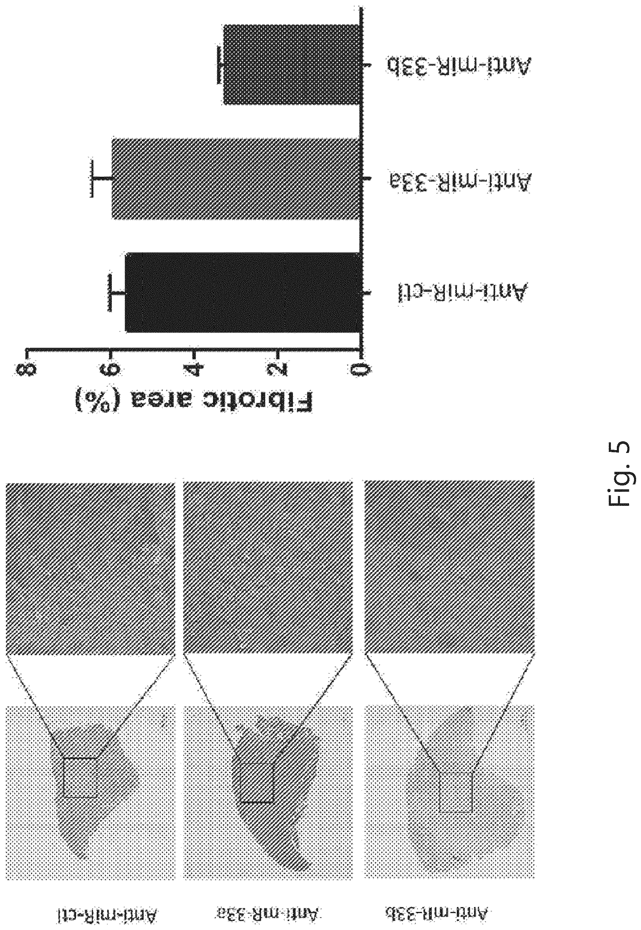

A change in muscle fibrosis in the anterior tibial muscle of the miR-33b knock-in/mdx mice caused by the administration of the miR-33b antisense oligonucleotide was measured by Masson's trichrome staining. As a result, as shown in Fig. 5, the miR-33b antisense oligonucleotide was found to have caused a significant, 0.58-fold reduction of the muscle fibrosis relative to the control. On the other hand, the miR-33a antisense oligonucleotide did not reduce the muscle fibrosis relative to the control. Thus, the miR-33b antisense oligonucleotide was found to suppress muscle fibrosis.

The Masson's trichrome staining method was as follows.

After deparaffinization, the nuclei were stained black with iron hematoxylin, and the cytoplasm was stained red with acid fuchsin, which is a small molecule. Collagen fibers were stained blue with aniline blue, which is a large molecule, and the fibrotic portion was quantified by image analysis (ImageJ).

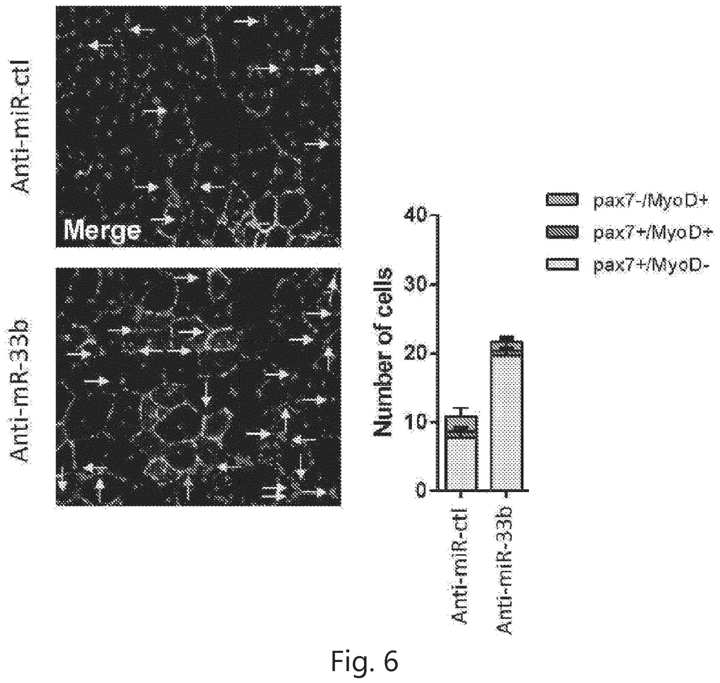

A change in the expression of Pax7/MyoD in muscle satellite cells in the anterior tibial muscle of the miR-33b knock-in/mdx mice caused by the administration of the miR-33b antisense oligonucleotide was measured by fluorescent immunostaining. As a result, as shown in Fig. 6, the miR-33b antisense oligonucleotide was found to have caused a significant increase in the Pax7+/MyoD-group relative to the control. Pax7+/MyoD- is a marker of hygiene cells in the resting stage. Since the miR-33b antisense oligonucleotide increases hygiene cells in the resting stage, it is thought to provide, upon occurrence of muscle injury, hygiene cells without depletion, to promote muscle regeneration. In the process of differentiation of hygiene cells into myoblasts and muscle cells, Pax7+/MyoD-becomes Pax7+/MyoD+. Therefore, a decrease in MyoD in hygiene cells indicates an increase in satellite cells, and an increase in MyoD indicates promotion of muscle differentiation.

The fluorescent immunostaining method was as follows.

After deparaffinization and subsequent antigen retrieval, double staining of Pax7 and MyoD (using Fab fragments) was carried out.

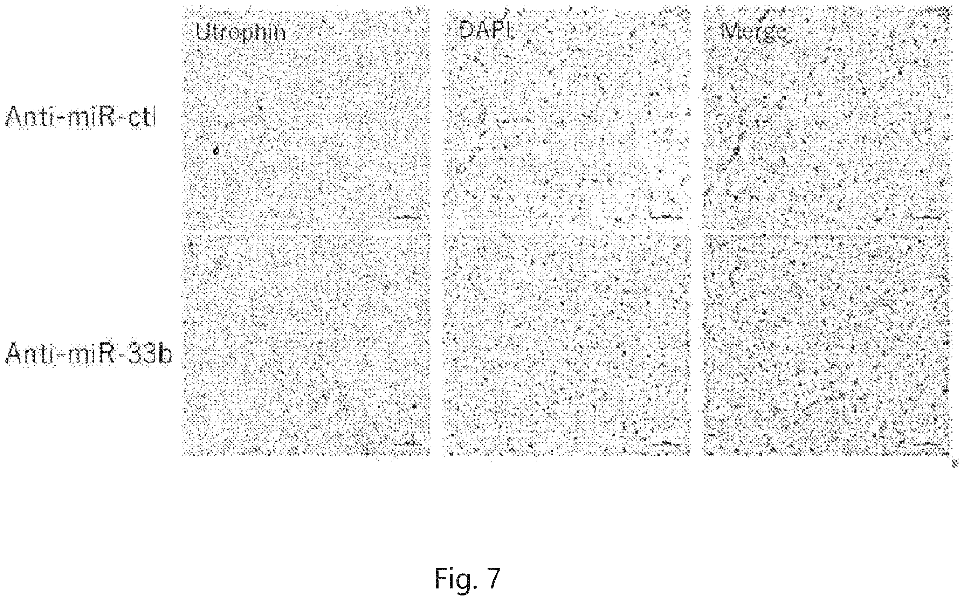

A change in the expression of Utrophin in the anterior tibial muscle of the miR-33b knock-in/mdx mice caused by the administration of the miR-33b antisense oligonucleotide was measured by the fluorescent immunostaining method using an anti-Utrophin antibody. As a result, as shown in Fig. 7, the miR-33b antisense oligonucleotide was found to have significantly increased the number of Utrophin-positive cells relative to the control.

The fluorescent immunostaining method was as follows.

After deparaffinization and subsequent antigen retrieval, reaction with a Utrophin antibody (primary antibody) was carried out, and then reaction using a fluorescent secondary antibody having cross-reactivity was carried out.

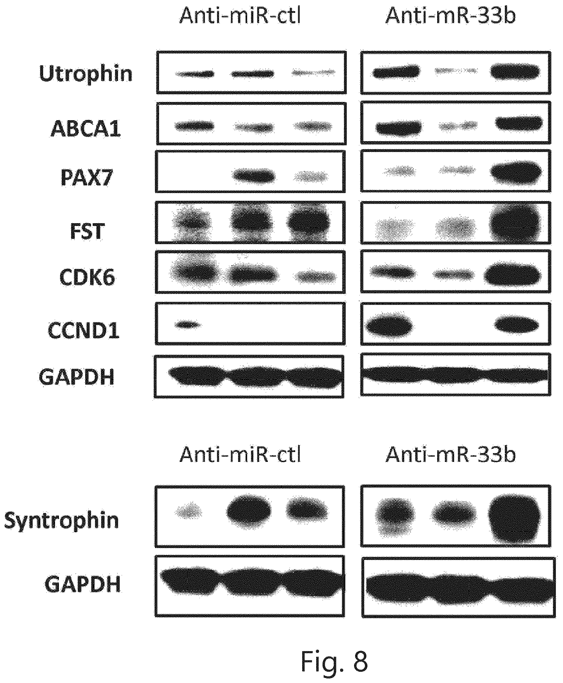

Changes in the expression of various proteins in the anterior tibial muscle of the miR-33b knock-in/mdx mice caused by the administration of the miR-33b antisense oligonucleotide were measured by the Western blotting method. As a result, as shown in Fig. 8, the miR-33b antisense oligonucleotide was found to have increased the protein expression of Utrophin, ABCA1, Pax7, Fst, Cdk6, CCND1, and syntrophin relative to the control.

The Western blotting method was as follows.

A membrane was adhered to the gel after SDS-PAGE, and voltage was applied to the separated proteins to transfer them to the membrane. The membrane was reacted with a primary antibody (an antibody against the target protein), and then with a secondary antibody labeled with the HRP enzyme. Thereafter, detection was carried out by the chemiluminescence method.

The antibodies used were as follows.
anti-Utrophin, Santa cruz 8A4
anti-ABCA1, Novus NB400-105
anti-Pax7, DSHB (Developmental Studies Hybridoma Bank)
anti-Cdk6, Cell Signal Technology CST#3136
anti-Follistatin, Santa cruz C-8
anti-GAPDH, Cell Signal Technology CST#2118
anti-syntrophin, Santa cruz D-7
anti-CCND1, Abcam ab92566

All mice were maintained in the Institute of Laboratory Animals, Graduate School of Medicine, Kyoto University, wherein the SPF (specific pathogen free) conditions were maintained. The present study was carried out with approval by the ethical committee of Graduate School of Medicine, Kyoto University.

In the present description, the singular is used in a manner in which it includes the plural, unless otherwise specified. In the present description, "or" means "and/or" unless otherwise specified. Further, the use of the term "including" and its other forms such as "includes" and "included" is not limiting. Further, terms such as "element" encompass elements containing one unit and elements containing more than one subunit, unless otherwise specified.

The section titles in the present description are used merely for the structural purpose, and should not be construed as limiting the subject matter described. All documents and parts thereof cited in the present application, including, but not limited to, patents, patent applications, articles, books, and papers, are hereby expressly incorporated in the present description by reference regarding the parts or entireties of the documents discussed herein.

Unless specific definitions are provided, the nomenclatures, procedures, and techniques utilized in connection with the analytical chemistry, synthetic organic chemistry, medical chemistry, and pharmaceutical chemistry described in the present description are those which are well known and commonly used in the art. Standard techniques may be used for the chemical syntheses and the chemical analyses used in the present description. Where permitted, all patents, applications, published applications, and other publications; GenBank accession numbers and related sequence information available through databases such as National Center for Biotechnology Information (NCBI) and other databases; and other data; mentioned throughout the present disclosure are incorporated by reference regarding the parts and the entireties of the documents discussed herein.

The present description is being filed along with a Sequence Listing in an electronic format. The information in the Sequence Listing described in the electronic format is incorporated herein by reference in its entirety.

## Claims

1. A prophylactic or therapeutic agent for a myopathy, comprising a miR-33b inhibitor as an effective component.

2. The prophylactic or therapeutic agent according to claim 1, wherein the miR-33b inhibitor is a miR-33b-level-reducing substance.

3. The prophylactic or therapeutic agent according to claim 2, wherein the miR-33b-level-reducing substance is nucleic acid.

4. The prophylactic or therapeutic agent according to claim 3, wherein the miR-33b-level-reducing substance is an antisense oligonucleotide, a siRNA, or a shRNA against miR-33b.

5. The prophylactic or therapeutic agent according to any one of claims 2 to 4, wherein the miR-33b-level-reducing substance has a miR-33b-level-reducing rate that is higher than the miR-33a-level-reducing rate.

6. The prophylactic or therapeutic agent according to any one of claims 2 to 5, wherein the miR-33b-level-reducing substance is an antisense oligonucleotide against miR-33b.

7. The prophylactic or therapeutic agent according to claim 6, wherein the antisense oligonucleotide against miR-33b
1) is composed of 7 to 20 nucleotide residues, and
2) has a base sequence which is not less than 90% complementary to an equal length portion of miR-33b having the base sequence of SEQ ID NO:1, wherein no mismatch is present at either the 9th or 10th nucleic acid base as counted from the 5'-end of the miR-33b base sequence of SEQ ID NO: 1. (SEQ ID NO: 1) GUGCAUUGCUGUUGCAUUGC

8. The prophylactic or therapeutic agent according to claim 7, wherein the antisense oligonucleotide against miR-33b has a nucleic acid base sequence having the sequence of AACAGCAATGCA (SEQ ID NO:5) (wherein C is optionally 5-methylcytosine (M)).

9. The prophylactic or therapeutic agent according to claim 8, wherein the antisense oligonucleotide is a modified oligonucleotide.

10. The prophylactic or therapeutic agent according to claim 9, wherein at least one nucleoside constituting the modified oligonucleotide contains a modified sugar.

11. The prophylactic or therapeutic agent according to claim 10, wherein the modified sugar is selected from the sugar moiety of AmNA: 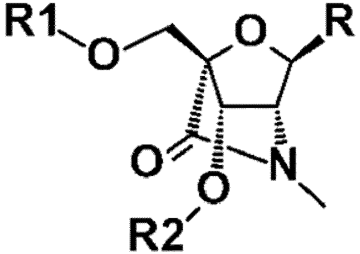 AmNA [wherein R is a nucleic acid base; and R1 and R2 are each independently a phosphate group that is optionally independently substituted].

12. The prophylactic or therapeutic agent according to claim 11, wherein the modified oligonucleotide is a modified oligonucleotide of the following formula:
Aas Ads Mas Ads Gas Cds Aas Ads Tas Gds Mas Ad (SEQ ID NO:8), wherein in the formula,
each nucleic acid base is represented according to the following symbol: A = adenine, T = thymine, G = guanine, C = cytosine, or M = 5-methylcytosine;
each sugar moiety is represented according to the following symbol: a = the sugar moiety of AmNA, or d = 2'-deoxyribose; and
each internucleoside bond is represented according to the following symbol: s = phosphorothioate.

13. The prophylactic or therapeutic agent according to claim 12, wherein the modified oligonucleotide is a modified oligonucleotide of the following formula:

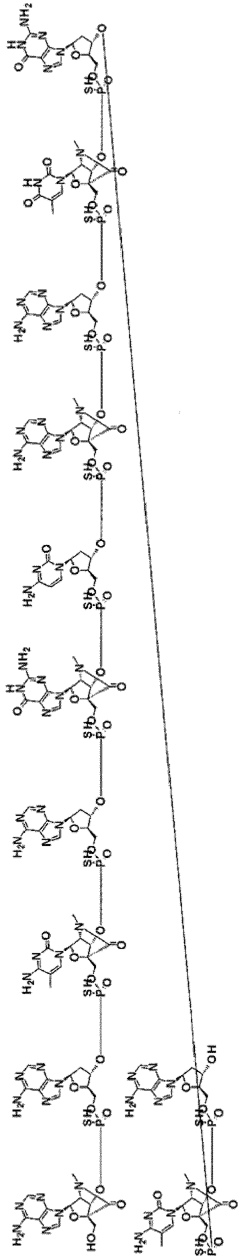

14. The prophylactic or therapeutic agent according to any one of claims 1 to 13, which produces a prophylactic or therapeutic effect on a myopathy by increasing the muscle fiber cross-sectional area.

15. The prophylactic or therapeutic agent according to any one of claims 1 to 13, which produces a prophylactic or therapeutic effect on a myopathy by promoting muscle differentiation.

16. The prophylactic or therapeutic agent according to any one of claims 1 to 15, wherein the myopathy is selected from the group consisting of neurogenic muscle atrophy, myogenic muscle atrophy, and a disease of the neuromuscular junction.

17. The prophylactic or therapeutic agent according to any one of claims 1 to 15, wherein the myopathy is selected from the group consisting of cachexia, sarcopenia, disuse atrophy, and hereditary spastic paraplegia.

18. The prophylactic or therapeutic agent according to claim 16, wherein the neurogenic muscle atrophy is selected from the group consisting of spinal muscular atrophy, spinal and bulbar muscular atrophy, and amyotrophic lateral sclerosis.

19. The prophylactic or therapeutic agent according to claim 16, wherein the myogenic muscle atrophy is selected from the group consisting of muscular dystrophy, congenital myopathy, inflammatory myopathy, and metabolic myopathy.

20. The prophylactic or therapeutic agent according to claim 16, wherein the disease of the neuromuscular junction is selected from the group consisting of myasthenia gravis, myasthenic syndrome, and congenital myasthenia.
